# EUROPEAN PATENT APPLICATION

(11) **EP 4 799 642 A1**
(43) Date of publication of application: **02.09.2026**
(21) Application number: 24881573.0
(22) Date of filing: 22.10.2024
(51) Int. Cl.: A61K 45/06, A61K 31/438, A61P 7/06

(54) **USE OF HYPOXIA-INDUCIBLE FACTOR-PROLYL HYDROXYLASE INHIBITOR (HIF-PHI) IN RARE ANEMIA**

(30) Priority: 24.10.2023 CN 202311391912
(71) Applicant: Kind Pharmaceutical, Hangzhou Zhejiang 311100 (CN)
(72) Inventor: DENG, Shaojiang, Hangzhou, Zhejiang 311100 (CN); LIU, Dong, Hangzhou, Zhejiang 311100 (CN); XIONG, Min, Hangzhou, Zhejiang 311100 (CN); LIU, Jiang, Hangzhou, Zhejiang 311100 (CN)
(74) Representative: Barrow, Nicholas Martin
(86) International application number: PCT/CN2024/126291
(87) International publication number: WO 2025/087208

(57) **Abstract**

The present application relates to use of a hypoxia-inducible factor-prolyl hydroxylase inhibitors (HIF-PHI) in rare anemia. Specifically disclosed in the present application is use of certain hypoxia-inducible factor-prolyl hydroxylase inhibitors (HIF-PHIs) in the treatment of anemia of myelodysplastic syndromes (MDS anemia), beta-thalassemia (β-thalassemia), and/or sickle cell disease (sickle cell anemia, SCD anemia).

## Description

### CROSS-REFERENCE TO RELATED APPLICATION

This application claims priority to Chinese Patent Application No. 202311391912.1 filed on October 24, 2023 and entitled with "use of hypoxia-inducible factor-prolyl hydroxylase inhibitor (HIF-PHI) in rare anemia", which is incorporated herein by reference in its entirety.

### TECHNICAL FIELD

The present application pertains to the medical field, and in particular, to the use of certain hypoxia-inducible factor-prolyl hydroxylase inhibitors (HIF-PHIs) in the treatment of rare anemias.

### BACKGROUND

The primary function of red blood cells in the human body is to adsorb oxygen inhaled from lungs onto hemoglobin within the red blood cells. The adsorbed oxygen is then transported via bloodstream to body's tissues, where it is used for cellular metabolism. Subsequently, carbon dioxide produced by the body's cells is adsorbed by the hemoglobin in the red blood cells and transported back through the bloodstream to the lungs, where it is expelled from the body so that a cycle of inhaling oxygen, transporting oxygen and carbon dioxide, and expelling carbon dioxide is completed. Normal hemoglobin levels in the human body can vary based on factors such as age and gender. For example, normal hemoglobin levels range from 14-18 g/dL in adult men and 12-16 g/dL in adult women. Erythropoietin (EPO) is a key hormone for maintaining homeostasis of red blood cells and hemoglobin in the human body. In healthy individuals, hemoglobin levels are negatively correlated with endogenous EPO. That is, a decrease in hemoglobin leads to an increase in endogenous EPO, which promotes hemoglobin production and red blood cell growth to compensate for the decrease in hemoglobin leads, thereby maintaining homeostasis.

A reduction in the absolute number of red blood cells or a decrease in hemoglobin levels can lead to anemia. Anemia not only severely impacts a patient's health and quality of life but, if left uncorrected, can even threaten a patient's life. Anemia encompasses many different types of diseases. Among them, anemia caused by chronic kidney disease (CKD) is one of the most common forms. It also includes various types of rare anemias that have long troubled patients and physicians, such as myelodysplastic syndrome-associated anemia (MDS anemia), β-thalassemia, and sickle cell disease (SCD anemia). The clinical manifestations and underlying biological mechanisms of common anemias like CKD anemia and rare anemias such as MDS anemia, β-thalassemia, and SCD anemia are completely different; consequently, the existing pharmacological treatment approaches for them are not entirely identical, which is entirely logical.

MDS anemia is a rare, heterogeneous clonal stem cell disorder characterized by dysplasia in myeloid cells, manifesting as ineffective hematopoiesis and dysplastic hematopoiesis, leading to single or multilineage cytopenias and a high risk of progression to acute myeloid leukemia. The global incidence of MDS anemia is approximately 2-12 per 100,000. Eighty percent of cases occur in individuals over 60 years of age, with a male predominance. Patients with MDS anemia can be categorized into lower-risk and higher-risk groups. Apart from red blood cell transfusions, the primary supportive treatment for anemia in MDS patients involves recombinant human erythropoietin (rhEPO)-type drugs; however, the response rate of MDS anemia to rhEPO is very low.

β-thalassemia is a common inherited chronic blood disorder clinically. Its pathogenesis involves mutations or deletions in β-globin gene of hemoglobin (Hb), leading to an imbalance in the quantities of α and β globin chains. This imbalance results in defective erythropoiesis or ineffective erythropoiesis, causing chronic anemia and complications in patients. The highest incidence rates are found in the Mediterranean region, Southeast Asia, and the Middle East. In China, the β-thalassemia patients are mainly distributed in southern regions such as Guangdong, Guangxi, Yunnan, Guizhou, Sichuan, Hong Kong, and Macau, with an average prevalence of about 2%. Among these, transfusion-dependent thalassemia (TDT), i.e., severe cases, often presents in infancy with chronic progressive hemolytic anemia, severely threatening the patient's quality of life and even life. Without treatment, severe β-thalassemia often leads to death before the age of five. Standard lifelong transfusion and iron chelation therapy are the mainstays for treating severe β-thalassemia; splenectomy is a palliative procedure, and hematopoietic stem cell transplantation is the only curative therapy. However, its application is limited by a lack of suitable donors, prohibitive medical costs, and limited medical resources. Recently, gene therapies have been approved for treating β-thalassemia, but they are extremely expensive, suitable for a very limited patient population, and their long-term safety remains unknown. More recently, luspatercept as a TGF-β targeting fusion protein erythroid maturation agent was approved for severe β-thalassemia, but a significant number of patients do not respond to this drug, and side effects such as arthralgia and bone pain can be severe. Severe thalassemia is classified as a rare disease in both China and the United States.

SCD anemia is another category of very severe, life-threatening inherited chronic blood disorder, predominantly affecting people of African descent and their descendants. There are approximately 100,000 patients in the United States and over 3 million globally. SCD anemia is also considered a rare disease in both China and the United States. This disease results from a mutation where the sixth amino acid of hemoglobin β-globin chain changes from hydrophilic, negatively charged glutamic acid to hydrophobic valine. This results in the physical aggregation/polymerization of hemoglobin in the patient's red blood cell cytoplasm after the bound oxygen molecules are exchanged in the tissues, causing the red blood cells to collapse into a sickle shape. Clinically, this manifests as severe hemolytic anemia, and because the deformed red blood cells block capillaries, it leads to local ischemia, pain, and multi-organ failure. This causes hemoglobin in the patient's red blood cell cytoplasm to physically aggregate/polymerize after releasing oxygen molecules in the tissues, leading to the collapse of red blood cells into a sickle shape. Clinically, this presents as severe hemolytic anemia, and the deformed red blood cells block capillaries, causing local ischemia, pain, and multi-organ failure. Pharmacological treatment for SCD anemia primarily involves hydroxyurea (HU). However, HU itself is a chemotherapeutic agent with certain myelosuppressive side effects, and long-term use may lead to leukemia. Additionally, a small molecule drug, voxelotor, recently received conditional approval from the US FDA but has not yet received final full approval. Its mechanism involves inserting into cavity of deoxygenated hemoglobin to prevent polymerization of the hemoglobin, but its clinical efficacy is limited, and it does not improve patient-reported outcomes (PRO).

Overall, for the aforementioned rare anemias-such as myelodysplastic syndrome-associated anemia (MDS anemia), β-thalassemia, and sickle cell disease (SCD anemia)-there are currently no safe and effective treatments available. Particularly no safe and effective small molecule drugs are available.

Therefore, there is a significant need for a safe and effective small molecule drug for the treatment of rare anemias, especially rare anemias such as MDS anemia, β-thalassemia, and SCD anemia.

### SUMMARY

To discover safe and effective small molecule drugs for the treatment of rare anemias, particularly those such as MDS anemia, β-thalassemia, and SCD anemia, the inventors of the present application conducted extensive research and investigation.

As one of the therapeutic options for common anemias like chronic kidney disease (CKD) anemia, oral hypoxia-inducible factor-prolyl hydroxylase inhibitors (HIF-PHIs) have gradually been approved by some regulatory agencies. As a novel class of small molecule drugs, the principle of HIF-PHIs in treating CKD anemia involves inhibiting hypoxia-inducible factor prolyl hydroxylase, thereby preventing degradation of HIF-α. This allows HIF-α to combine with HIF-β, forming a transcriptionally active HIF dimer. This transcription factor then initiates expression of multiple downstream target genes, including increasing the production of endogenous EPO, thus achieving an effect similar to recombinant erythropoietin in treating CKD anemia. According to published reports, HIF-PHIs that have entered clinical trials for the treatment of renal anemia in CKD include Roxadustat, Daprodustat, Vadadustat, Molidustat, Enarodustat, Desidustat, HIF117 (SSS17), HEC53856, DDO-3055, and FG-2216. Even so, not every HIF-PHI can be truly safe and effective in treating CKD anemia. For instance, to date, in the United States, only Daprodustat has succeeded in pivotal Phase 3 clinical trials for the indication of dialysis-dependent CKD anemia, and its New Drug Application (NDA) was approved by the FDA. Daprodustat's pivotal Phase 3 clinical trials for non-dialysis CKD anemia in the US were not successful, and its NDA for that indication was not approved by the FDA. Similarly, Roxadustat's pivotal Phase 3 clinical trials for both dialysis and non-dialysis CKD anemia in the US were not successful, and its NDA was ultimately not approved by the FDA. Therefore, even though many molecules are considered HIF-PHIs, their ability to be proven safe and effective in CKD anemia is not self-evident.

Based on the mechanism of HIF-PHIs stabilizing HIF, activating EPO, and consequently elevating EPO levels, HIF-PHIs were once considered ideal replacements for injectable recombinant erythropoietin (rEPO) in the treatment of CKD anemia. For this reason, all currently reported HIF-PHIs are being developed for the treatment of CKD anemia. Only Roxadustat has been tested for the treatment of MDS anemia; however, its efficacy in Phase 3 clinical trials for MDS was found to be not significantly different from placebo, resulting in failure. It was reported on news dated May 5, 2023: https://www.biospace.com/article/releases/fibrogen-announces-results-for-matterhorn-a-phase-3-clinical-study-of-roxadustat-for-the-treatment-of-anemia-in-patients-with-myelodysplastic-syndromes-mds-study-did-not-meet-primary-endpoint/). Other HIF-PHIs are very rarely developed for rare anemias, as the published biological mechanisms of rare anemias such as MDS anemia, β-thalassemia, and SCD anemia are distinctly different from those of CKD anemia.

The failures of some HIF-PHIs mentioned above in pivotal Phase 3 clinical trials for CKD anemia in the US and subsequent NDA rejections, contrasted with the success of Daprodustat alone in pivotal Phase 3 clinical trials for dialysis-dependent CKD anemia and its subsequent FDA approval, demonstrate that despite ostensibly similar mechanisms of action, the safety and efficacy profiles of HIF-PHIs in the most common form, CKD anemia, are entirely different. This means that the safety and efficacy of these HIF-PHIs in CKD anemia are not obvious. Moreover, their safety and efficacy in rare anemias such as myelodysplastic syndrome-associated anemia (MDS anemia), β-thalassemia, and sickle cell disease (SCD, also referred to as SCD anemia) are even less predictable. Furthermore, the failure of Roxadustat in pivotal Phase 3 clinical trials for MDS anemia in 2023 has made the application of various HIF-PHIs in rare anemias like MDS anemia, β-thalassemia, and SCD anemia even more difficult to predict.

However, unexpectedly, through in-depth research, the inventors of the present application discovered that an indolizine derivative (e.g., the compound synthesized in Example 24 of WO 2018205928) effectively increased red blood cell count, hemoglobin levels, and hematocrit in mouse models of MDS anemia, β-thalassemia, and SCD anemia, thereby ameliorating MDS anemia, β-thalassemia, and SCD anemia. Even more surprisingly, the aforementioned indolizine derivative exhibited remarkable efficacy in mouse models of MDS anemia, β-thalassemia, and sickle cell disease (SCD) anemia, respectively. Therefore, it exhibits potential for treating MDS anemia, β-thalassemia, and SCD anemia.

Furthermore, through further in-depth investigation, the inventors of the present application discovered that hypoxia-inducible factor-prolyl hydroxylase inhibitor (HIF-PHI) molecules other than Roxadustat may possess therapeutic functions for MDS anemia or SCD anemia. Additionally, HIF-PHI molecules including Roxadustat may possess therapeutic functions for β-thalassemia.

Therefore, a first aspect of the present application provides use of a hypoxia-inducible factor-prolyl hydroxylase inhibitor (HIF-PHI) in the manufacture of a medicament for treating a rare anemia.

A second aspect of the present application provides use of a hypoxia-inducible factor-prolyl hydroxylase inhibitor (HIF-PHI) in the manufacture of a medicament for increasing red blood cell count, hemoglobin levels, and/or hematocrit in a rare anemia.

A third aspect of the present application provides a method for treating a rare anemia, comprising administering a therapeutically effective amount of a hypoxia-inducible factor-prolyl hydroxylase inhibitor (HIF-PHI) to a patient in need thereof.

A fourth aspect of the present application provides a method for increasing red blood cell count, hemoglobin levels, and/or hematocrit in a rare anemia, comprising administering a therapeutically effective amount of a hypoxia-inducible factor-prolyl hydroxylase inhibitor (HIF-PHI) to a patient in need thereof.

A fifth aspect of the present application provides a hypoxia-inducible factor-prolyl hydroxylase inhibitor (HIF-PHI) for use in treating a rare anemia.

A sixth aspect of the present application provides use of a hypoxia-inducible factor-prolyl hydroxylase inhibitor (HIF-PHI) for treating a rare anemia.

### DETAIL DESCRIPTION

### I. Hypoxia-inducible Factor-Prolyl Hydroxylase Inhibitors (HIF-PHIs)

In various aspects of the present application and various embodiments of these aspects, the hypoxia-inducible factor-prolyl hydroxylase inhibitor can be various hypoxia-inducible factor-prolyl hydroxylase inhibitor compounds known in the art.

In some preferred embodiments of the present application, the hypoxia-inducible factor-prolyl hydroxylase inhibitor can be an HIF-PHI compound, or a pharmaceutically acceptable salt thereof, that has been approved for clinical use or is undergoing clinical trials. For example, the HIF-PHI can be selected from the group consisting of Roxadustat, Daprodustat, Vadadustat, Molidustat, Enarodustat, Desidustat, HIF117 (SSS17), HEC53856, and DDO-3055; or the HIF-PHI can be selected from the group consisting of Daprodustat, Vadadustat, Molidustat, Enarodustat, Desidustat, HIF117 (SSS17), HEC53856, and DDO-3055.

In some embodiments, the hypoxia-inducible factor-prolyl hydroxylase inhibitor (HIF-PHI) is Roxadustat or a pharmaceutically acceptable salt thereof.

In some embodiments, the hypoxia-inducible factor-prolyl hydroxylase inhibitor (HIF-PHI) is Daprodustat or a pharmaceutically acceptable salt thereof.

In some embodiments, the hypoxia-inducible factor-prolyl hydroxylase inhibitor (HIF-PHI) is Vadadustat or a pharmaceutically acceptable salt thereof.

In some embodiments, the hypoxia-inducible factor-prolyl hydroxylase inhibitor (HIF-PHI) is Molidustat or a pharmaceutically acceptable salt thereof.

In some embodiments, the hypoxia-inducible factor-prolyl hydroxylase inhibitor (HIF-PHI) is Enarodustat or a pharmaceutically acceptable salt thereof.

In some embodiments, the hypoxia-inducible factor-prolyl hydroxylase inhibitor (HIF-PHI) is Desidustat or a pharmaceutically acceptable salt thereof.

In some embodiments, the hypoxia-inducible factor-prolyl hydroxylase inhibitor (HIF-PHI) is HIF117 (SSS17) or a pharmaceutically acceptable salt thereof.

In some embodiments, the hypoxia-inducible factor-prolyl hydroxylase inhibitor (HIF-PHI) is HEC53856 or a pharmaceutically acceptable salt thereof.

In some embodiments, the hypoxia-inducible factor-prolyl hydroxylase inhibitor (HIF-PHI) is DDO-3055 or a pharmaceutically acceptable salt thereof.

In some embodiments, the hypoxia-inducible factor-prolyl hydroxylase inhibitor (HIF-PHI) is a compound of Formula I disclosed in WO2018205928, or a pharmaceutically acceptable salt thereof.

In some embodiments, the hypoxia-inducible factor-prolyl hydroxylase inhibitor (HIF-PHI) is any mixture of the aforementioned HIF-PHIs.

In the present application, unless otherwise specified, any reference to or designation of a compound or its pharmaceutically acceptable salt by structural formula, name, or code, such as "compound of Formula I" (synonymous with "compound represented by general Formula I"), "Roxadustat", or "(6'-hydroxy-8'-oxo-3'-phenyl-8'H-spiro[cyclopentane-1,5'-indolizine]-7'-carbonyl)glycine", and the like, encompasses various forms of these compounds, including tautomers thereof, optical isomers thereof, geometric isomers thereof, or mixtures of these isomers (e.g., racemic mixtures), and isotopically modified compounds thereof, solvates or hydrates thereof, as well as various solid forms thereof (e.g., various crystalline or amorphous forms thereof).

The term "optical isomer" means that when a compound has one or more chiral centers, each chiral center can exist in the R configuration or the S configuration, and the various isomers constituted thereby are optical isomers. Optical isomers include all diastereomers, enantiomers, mesomers, racemates, or mixtures thereof. For example, optical isomers can be separated by chiral chromatography or by chiral synthesis.

The term "geometric isomer" means that when a double bond exists in a compound, the compound can exist as a cis isomer, trans isomer, E isomer, and Z isomer. Geometric isomers include cis isomers, trans isomers, E isomers, Z isomers, or mixtures thereof.

The term "tautomer" refers to isomers produced by rapid migration of a certain atom within a molecule between two positions. Those skilled in the art can understand that tautomers can interconvert and may reach an equilibrium state in a certain state to achieve coexisting. For example, "compound of Formula I" described herein also encompasses any tautomer of the compound of general Formula I. Specifically, the inventors have found that the compound of Formula I may exist as the following tautomers I-a, I-b, I-c, or I-d:

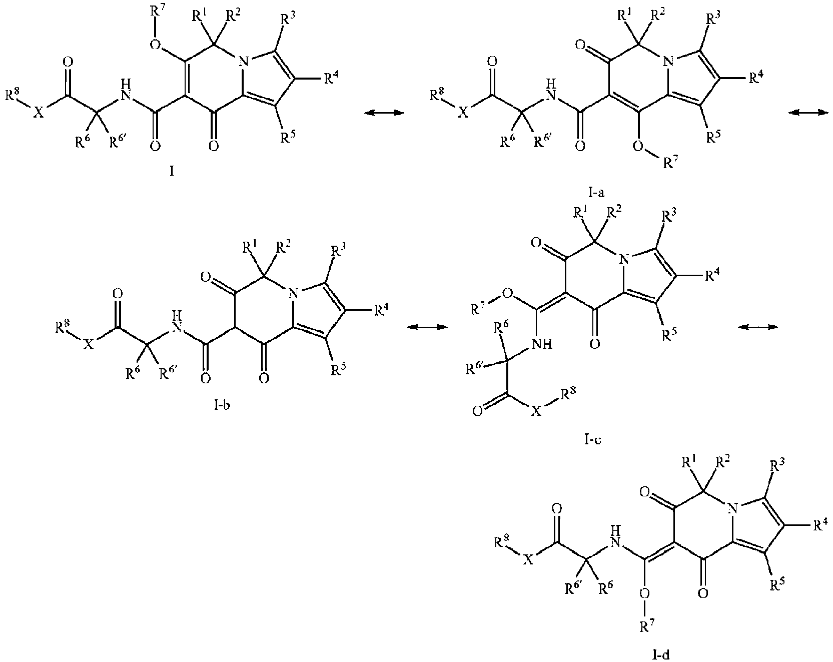

The term "an isotopically modified compound" refers to a compound obtained by replacing any atom in the compound with its isotopic atom. "Isotopically modified compounds" in the present application include all pharmaceutically acceptable isotopically modified compounds of the compound, wherein one or more atoms are replaced by atoms having the same atomic number as those usually found in nature, but with different atomic masses or mass numbers.

Examples of isotopes suitable for inclusion in the compounds of the present application include isotopes of hydrogen, such as ²H (D) and ³H (T); isotopes of carbon, such as ¹¹C, ¹³C, and ¹⁴C; isotopes of chlorine, such as ³⁶Cl; isotopes of fluorine, such as ¹⁸F; isotopes of iodine, such as ¹²³I and ¹²⁵I; isotopes of nitrogen, such as ¹³N and ¹⁵N; isotopes of oxygen, such as ¹⁵O, ¹⁷O, and ¹⁸O; and isotopes of sulfur, such as ³⁵S.

Certain isotopically-labelled compounds of formula (I), for example, those incorporating a radioactive isotope, are useful in drug and/or substrate tissue distribution studies. The radioactive isotopes deuterium, i.e. ²H, tritium, i.e. ³H, and carbon-14, i.e. ¹⁴C, are particularly useful for this purpose in view of their ease of incorporation and ready means of detection.

Substitution with heavier isotopes such as deuterium, i.e. ²H, or tritium, i.e. ³H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-modified compounds of formula (I) can generally be prepared by conventional techniques known to those skilled in the art or by processes analogous to those described in the accompanying Examples and Preparations using an appropriate isotopicallylabeled reagents in place of the non-labeled reagent previously employed.

Certain compounds of the present application may exist in unsolvated form as well as solvated forms, including hydrated forms.

Certain compounds of the present application may exist in different crystalline or amorphous forms, and all such forms are included within the scope of the present application.

In the present application, "pharmaceutically acceptable salt" refers to inorganic or organic acid addition salts, or organic or inorganic base addition salts of the compound that are suitable for in vivo use in mammals (i.e., safe and effective for use). These salts may be prepared in situ during final isolation and purification of the compound, or by separately reacting purified compound in its free form with a suitable organic or inorganic acid or base and isolating the salt thus formed. Typical salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, glucoheptonate, lactobionate, lauryl sulfate, and the like. These salts may include those salts based on cations such as alkali and alkaline earth metals (e.g., sodium, lithium, potassium, calcium, magnesium, etc.), as well as non-toxic ammonium, quaternary ammonium, and amine cations (including, but not limited to, ammonium, tetramethylammonium, tetraethylammonium, methylamine, dimethylamine, trimethylamine, triethylamine, ethylamine, etc.).

In some preferred embodiments of the present application, the hypoxia-inducible factor-prolyl hydroxylase inhibitor (HIF-PHI) is a compound of Formula I or a pharmaceutically acceptable salt thereof disclosed in WO2018205928:

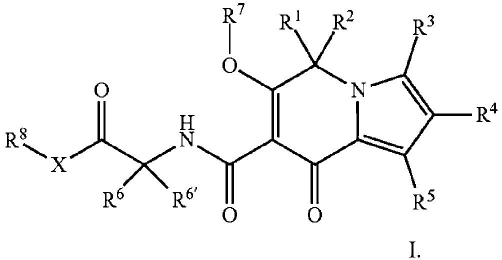

In some embodiments, in the compound represented by general Formula I:
R¹ and R² are independently selected from the group consisting of cyano, alkyl, heterocyclyl, alkenyl, alkynyl, aryl, heteroaryl, and acyl; wherein the aforementioned alkyl, heterocyclyl, alkenyl, alkynyl, aryl, heteroaryl, and acyl are optionally substituted by one or more substituents independently selected from the group consisting of halogen, cyano, hydroxyl, amino, carboxyl, acyl, alkyl, heterocyclyl, alkenyl, alkynyl, aryl, heteroaryl, =O, =S, SH, R¹⁰O-, R¹⁰S-, R¹⁰ (O=)S-, and R¹⁰ (O=)₂S-, wherein R¹⁰ is alkyl, heterocyclyl, alkenyl, alkynyl, aryl, or heteroaryl; or R₁ and R₂ are taken together to form a ring;
R³ is selected from the group consisting of alkyl, heterocyclyl, alkenyl, alkynyl, aryl, and heteroaryl;
R⁴ and R⁵ are hydrogen;
R⁶ and R⁶' are hydrogen;
R⁷ is hydrogen;
R⁸ is selected from the group consisting of hydrogen and alkyl; and
X is an oxygen atom.

In some embodiments, in the compound represented by general Formula I:
R¹ and R² are independently selected from the group consisting of cyano, C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, heteroaryl containing 5 to 14 ring atoms, C₁-C₁₂ acyclic alkyl-C(=O)-, and C₂-C₁₂ acyclic alkenyl-C(=O)-; wherein the aforementioned C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, heteroaryl containing 5 to 14 ring atoms, C₁-C₁₂ acyclic alkyl-C(=O)-, C₂-C₁₂ acyclic alkenyl-C(=O)- are optionally substituted by 1 to 3 substituents independently selected from the group consisting of hydroxyl, halogen, cyano, amino, carboxyl, C₁-C₆ acyclic alkyl, C₃-C₈ cycloalkyl, C₂-C₆ acyclic alkenyl-, C₂-C₆ acyclic alkynyl-, C₃-C₈ cycloalkyl, C₃-C₈ cycloalkenyl, C₆-C₁₄ aryl, heteroaryl containing 5 to 14 ring atoms, C₁-C₆ acyclic alkyl-O-, C₃-C₈ cycloalkyl-O-, C₂-C₆ acyclic alkenyl-O-, C₂-C₆ acyclic alkynyl-O-, C₃-C₈ cycloalkenyl-O-, C₆-C₁₄ aryl-O-, heteroaryl containing 5 to 14 ring atoms -O-, C₁-C₆ acyclic alkyl-S-, C₃-C₈ cycloalkyl-S-, C₂-C₆ acyclic alkenyl-S-, C₂-C₆ acyclic alkynyl-S-, C₃-C₈ cycloalkenyl-S-, C₆-C₁₄ aryl-S-, heteroaryl containing 5 to 14 ring atoms -S-, heterocycloalkyl containing 3 to 8 ring atoms, heterocycloalkenyl containing 3 to 8 ring atoms, =O, =S, SH, CF₃, -CO₂C₁-C₆ acyclic alkyl, C₁-C₆ acyclic alkyl-S-, C₁-C₆ acyclic alkyl(O=)S-, and C₁-C₆ acyclic alkyl(O=)₂S-; or
R¹ and R² are taken together to form an optionally substituted cycloalkane ring, cycloalkene ring, heterocycloalkane ring, or heterocycloalkene ring containing 3-8 ring atoms;
R³ is selected from the group consisting of C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, and heteroaryl containing 5 to 14 ring atoms; wherein the aforementioned C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, and heteroaryl containing 5 to 14 ring atoms are optionally substituted by 1 to 3 substituents independently selected from the group consisting of hydroxyl, halogen, cyano, amino, carboxyl, C₁-C₆ acyclic alkyl, C₃-C₈ cycloalkyl, C₂-C₆ acyclic alkenyl-, C₂-C₆ acyclic alkynyl-, C₃-C₈ cycloalkyl, C₃-C₈ cycloalkenyl, C₆-C₁₄ aryl, heteroaryl containing 5 to 14 ring atoms, C₁-C₆ acyclic alkyl-O-, C₃-C₈ cycloalkyl-O-, C₂-C₆ acyclic alkenyl-O-, C₂-C₆ acyclic alkynyl-O-, C₃-C₈ cycloalkenyl-O-, C₆-C₁₄ aryl-O-, heteroaryl containing 5 to 14 ring atoms -O-, C₁-C₆ acyclic alkyl-S-, C₃-C₈ cycloalkyl-S-, C₂-C₆ acyclic alkenyl-S-, C₂-C₆ acyclic alkynyl-S-, C₃-C₈ cycloalkenyl-S-, C₆-C₁₄ aryl-S-, heteroaryl containing 5 to 14 ring atoms -S-, heterocycloalkyl containing 3 to 8 ring atoms, heterocycloalkenyl containing 3 to 8 ring atoms, =O, =S, SH, CF₃, -CO₂C₁-C₆ acyclic alkyl, C₁-C₆ acyclic alkyl-S-, C₁-C₆ acyclic alkyl(O=)S-, and C₁-C₆ acyclic alkyl(O=)₂S-;
R⁴ and R⁵ are hydrogen;
R⁶ and R⁶' are hydrogen;
R⁷ is hydrogen;
R⁸ is selected from the group consisting of hydrogen, C₁-C₁₂ acyclic alkyl, and C₃-C₈ cycloalkyl; wherein the C₁-C₁₂ acyclic alkyl, and C₃-C₈ cycloalkyl are optionally substituted by 1 to 3 substituents independently selected from the group consisting of hydroxyl, halogen, cyano, amino, carboxyl, C₁-C₆ acyclic alkyl, C₃-C₈ cycloalkyl, C₂-C₆ acyclic alkenyl-, C₂-C₆ acyclic alkynyl-, C₃-C₈ cycloalkyl, C₃-C₈ cycloalkenyl, C₆-C₁₄ aryl, heteroaryl containing 5 to 14 ring atoms, C₁-C₆ acyclic alkyl-O-, C₃-C₈ cycloalkyl-O-, C₂-C₆ acyclic alkenyl-O-, C₂-C₆ acyclic alkynyl-O-, C₃-C₈ cycloalkenyl-O-, C₆-C₁₄ aryl-O-, and heteroaryl containing 5 to 14 ring atoms -O-; and
X is an oxygen atom.

In some preferred embodiments of the present application, in the compound represented by Formula I, R¹ and R² are independently selected from the group consisting of cyano and unsubstituted C₁-C₆ acyclic alkyl; or R¹ and R² are taken together to form an optionally substituted cycloalkane ring or heterocycloalkane ring containing 3-8 ring atoms.

In some preferred embodiments of the present application, in the compound represented by Formula I, R¹ and R² are independently selected from the group consisting of methyl and ethyl; or R¹ and R² are taken together to form a cyclopropane ring, a cyclobutane ring, a cyclopentane ring, a cyclohexane ring, a methylcyclohexane ring, a cycloheptane ring, a tetrahydrofuran ring, or a tetrahydropyran ring.

In some preferred embodiments of the present application, in the compound represented by Formula I, R¹ and R² are independently selected from the group consisting of cyano and unsubstituted C₁-C₆ acyclic alkyl.

In some preferred embodiments of the present application, in the compound represented by Formula I, R¹ and R² are independently selected from the group consisting of methyl and ethyl.

In some preferred embodiments of the present application, in the compound represented by Formula I, R¹ and R² are taken together to form an optionally substituted heterocycloalkane ring containing 3-8 ring atoms.

In some preferred embodiments of the present application, in the compound represented by Formula I, R¹ and R² are taken together to form a tetrahydrofuran ring or a tetrahydropyran ring.

In some preferred embodiments of the present application, in the compound represented by Formula I, R¹ and R² are taken together to form an optionally substituted cycloalkane ring containing 3-8 ring atoms.

In some preferred embodiments of the present application, in the compound represented by Formula I, R¹ and R² are taken together to form a cyclopropane ring, a cyclobutane ring, a cyclopentane ring, a cyclohexane ring, a methylcyclohexane ring, or a cycloheptane ring.

In some preferred embodiments of the present application, in the compound represented by Formula I, R³ is selected from the group consisting of C₁-C₆ acyclic alkyl, C₆-C₁₄ aryl, C₃-C₈ cycloalkyl, and heteroaryl containing 5 to 14 ring atoms; wherein the C₁-C₆ acyclic alkyl, C₆-C₁₄ aryl, and C₃-C₈ cycloalkyl are optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of methyl, ethyl, propyl, butyl, phenyl, benzyl, tolyl, methoxyphenyl, chlorophenyl, fluorophenyl, bromophenyl, cyclopropyl, cyclobutyl, cyclopentyl, cyclohexyl, and pyridyl.

In some preferred embodiments of the present application, in the compound represented by Formula I, R³ is C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of methyl, ethyl, propyl, and butyl.

In some preferred embodiments of the present application, in the compound represented by Formula I, R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of phenyl, benzyl, tolyl, methoxyphenyl, chlorophenyl, fluorophenyl, and bromophenyl, preferably phenyl.

In some preferred embodiments of the present application, in the compound represented by Formula I, R³ is C₃-C₈ cycloalkyl; wherein the C₃-C₈ cycloalkyl is optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of cyclopropyl, cyclobutyl, cyclopentyl, and cyclohexyl.

In some preferred embodiments of the present application, in the compound represented by Formula I, R³ is heteroaryl containing 5 to 14 ring atoms.

In some preferred embodiments of the present application, in the compound represented by Formula I, R³ is pyridyl.

In some preferred embodiments of the present application, in the compound represented by Formula I, R⁸ is selected from the group consisting of hydrogen and C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R⁸ is selected from the group consisting of hydrogen, methyl, ethyl, propyl, butyl, and pentyl.

In some preferred embodiments of the present application, in the compound represented by Formula I, R⁸ is hydrogen.

In some preferred embodiments of the present application, in the compound represented by Formula I, R⁸ is C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R⁸ is selected from the group consisting of methyl, ethyl, propyl, butyl, and pentyl.

In some preferred embodiments of the present application, in the compound represented by Formula I, R¹ and R² are taken together to form an optionally substituted cycloalkane ring containing 3-8 ring atoms; R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl; and R⁸ is selected from the group consisting of hydrogen and C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R¹ and R² are each independently selected from C₁-C₆ acyclic alkyl, wherein the C₁-C₆ acyclic alkyl is optionally substituted by 1 to 3 substituents of C₁-C₆ acyclic alkyl; or R¹ and R² are taken together to form a cyclopropane ring, a cyclobutane ring, a cyclopentane ring, a cyclohexane ring, a methylcyclohexane ring, a cycloheptane ring, or a tetrahydropyran ring; R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl; and R⁸ is selected from the group consisting of hydrogen and C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R¹ and R² are taken together to form a cyclopropane ring, a cyclobutane ring, a cyclopentane ring, a cyclohexane ring, a methylcyclohexane ring, or a cycloheptane ring; R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl; and R⁸ is hydrogen.

In some particularly preferred embodiments of the present application, the compound represented by Formula I is (6'-hydroxy-8'-oxo-3'-phenyl-8'H-spiro[cyclopentane-1,5'-indolizine]-7'-carbonyl)glycine. That is, the compound is those where R¹ and R² together with a carbon atom to which they are attached form a cyclopentane ring (meaning R¹ and R² are - CH₂CH₂CH₂CH₂-), R³ is phenyl, R⁴ and R⁵ are hydrogen, R⁶ and R⁶' are hydrogen, R⁷ is hydrogen, R⁸ is hydrogen, and X is oxygen.

In the various preferred embodiments above, the preferred options for each substituent can be combined with each other, and all such combinations are within the scope of the present application.

To avoid ambiguity, the definitions of the terms used herein are given below. Unless otherwise stated, the meanings of the terms used herein are as follows.

The term "hydroxy" refers to -OH.

The term "halogen" or "halo" refers to -F, -Cl, -Br, or -I.

The term "amino" refers to -NH₂.

The term "cyano" refers to -CN.

The term "carboxy" refers to -C(=O)OH.

The term "substituted" means that one or more (preferably 1 to 5, more preferably 1 to 3) hydrogen atoms in a group are independently replaced by a corresponding number of substituents.

The term "independently" means that when the number of substituents is more than one, these substituents may be the same or different.

The term "optional" or "optionally" means that the event described therein may or may not occur. For example, an "optionally substituted" group means that the group may be unsubstituted or substituted.

The term "heteroatom" as used herein refers to oxygen (O), nitrogen (N), or S(O)ₘ in which m may be 0, 1 or 2, i.e. a sulfur atom S, or a sulfoxide group SO, or a sulfonyl group S(O)₂).

The term "alkyl" refers to a group formed by removing a hydrogen atom at any carbon atom from a saturated hydrocarbon consisting solely of two elements, C and H. The "alkyl group" described herein includes an acyclic alkyl group such as a linear alkyl group or a branched alkyl group; and a cycloalkyl group such as a monocyclic alkyl group, a spirocycloalkyl group, a fused cycloalkyl group, or a bridged cycloalkyl group. Preferably, in the present application, the "alkyl" refers to an acyclic alkyl group. The alkyl group may be unsubstituted or substituted.

The "alkyl" as used herein includes an optionally substituted acyclic alkyl group which preferably has from 1 to 20 carbon atoms, more preferably from 1 to 12 carbon atoms, and most preferably from 1 to 6 carbon atoms; for example, methyl, ethyl, n-propyl, isopropyl, n-butyl, sec-butyl, isobutyl, tert-butyl, n-pentyl, n-hexyl, chloromethyl, fluoroethyl, trifluoromethyl or 1,1,1-trifluoroethyl and the like.

The "alkyl" as used herein also includes an optionally substituted cycloalkyl (e.g., C3-C20 cycloalkyl or C3-C12 cycloalkyl or C3-C8 cycloalkyl), for example, cyclopropyl, cyclobutyl, cyclopentyl, cyclohexyl, cycloheptyl, cyclooctyl, decalinyl, norbornyl, adamantyl, fluorocyclopropyl, 2-iodocyclobutyl, 2,3-dimethyl cyclopentyl, 2,2-dimethoxycyclohexyl and 3-phenylcyclopentyl and the like.

The "C1-C6 acyclic alkyl", also known as "lower acyclic alkyl", is a subset of alkyl which refers to a linear or branched alkyl group having from 1 to 6 carbon atoms, including, for example, methyl, ethyl, n-propyl, isopropyl, n-butyl, sec-butyl, isobutyl, tert-butyl, n-pentyl, n-hexyl, and the like.

The "alkyl" as used herein is optionally substituted by one or more substituents, wherein the substituents are independently selected from the group consisting of halo, cyano, nitro (-NO₂), hydroxy, amino, carboxy, acyl, alkyl, heterocyclyl, alkenyl, alkynyl, aryl, heteroaryl, =O, =S, -SH, R¹⁶O-, R¹⁶S-, R¹⁶(O=)S-, and R¹⁶(O=)₂S-, wherein R¹⁶ is alkyl, heterocyclyl, alkenyl, alkynyl, aryl or heteroaryl.

Preferably, the "alkyl" as used herein is optionally substituted with from 1 to 3 substituents, wherein the substituents are independently selected from the group consisting of hydroxy, halo, nitro, cyano, amino, carboxy, C1-C6 acyclic alkyl, C3-C8 cycloalkyl, C2-C6 acyclic alkenyl-, C2-C6 acyclic alkynyl-, C3-C8 cycloalkyl, C3-C8 cycloalkenyl, C6-C14 aryl, heteroaryl having 5 to 14 ring members, C1-C6 acyclic alkyl-O-, C3-C8 cycloalkyl-O-, C2-C6 acyclic alkenyl-O-, C2-C6 acyclic alkynyl-O-, C3-C8 cycloalkenyl-O-, C6-C14 aryl-O-, heteroaryl-O- having 5 to 14 ring members, C1-C6 acyclic alkyl-S-, C3-C8 cycloalkyl-S-, C2-C6 acyclic alkenyl-S-, C2-C6 acyclic alkynyl-S-, C3-C8 cycloalkenyl-S-, C6-C14 aryl-S-, heteroaryl-S- having 5 to 14 ring members, heterocycloalkyl having 3 to 8 ring members, heterocycloalkenyl having 3 to 8 ring members, =O, =S, -SH, -CF₃, -CO₂C₁-C₆ acyclic alkyl group, C1-C6 acyclic alkyl-S-, C1-C6 acyclic alkyl (O=)S- and C1-C6 acyclic alkyl (O=)₂S-.

The term "alkenyl" refers to a group formed by removing a hydrogen atom at any carbon atom from a hydrocarbon that consists of only two elements, C and H, and which contains one or more carbon-carbon double bonds without carbon-carbon triple bonds or aromatic bonds. The "alkenyl" as used herein includes an acyclic alkenyl group such as a linear or branched alkenyl group; and also includes a cyclic alkenyl group such as a monocycloalkenyl group, a spirocycloalkenyl group, a fused cycloalkenyl group or a bridged cycloalkenyl group. The alkenyl group may be unsubstituted or substituted.

The "alkenyl" as used herein includes an optionally substituted acyclic alkenyl group, preferably having from 2 to 20 carbon atoms, more preferably from 2 to 12 carbon atoms, most preferably from 2 to 6 carbon atoms; for example, vinyl, 1-propenyl, 2-propenyl, 1-butenyl, 2-butenyl, isobutenyl, 1-pentenyl, 2-pentenyl, isopentenyl, 1-hexenyl, 2-hexenyl, 3-hexenyl, isohexenyl, 1-heptenyl, 2-heptenyl, 3-heptenyl, 1-octenyl, 2-octenyl, 3-octenyl, 4-octenyl, 1-nonenyl, 1-decenyl, 1-undecenyl, 1-dodecenyl, and the like.

The "alkenyl" as used herein also includes an optionally substituted cycloalkenyl (e.g., C3-C20 cycloalkenyl or C3-C12 cycloalkenyl or C3-C8 cycloalkenyl), for example, cyclobutenyl, cyclopentenyl, cyclohexenyl, cycloheptenyl, cyclobutadienyl, cyclopentadienyl, cycloheptatrienyl, and the like.

The "alkenyl" as used herein is optionally substituted by one or more (e.g., 1-3) substituents, wherein the choice and preference of the substituents are the same as those for the "alkyl".

The term "alkynyl" refers to a group formed by removing a hydrogen atom at any carbon atom from a hydrocarbon that consists of only two elements C and H and which contains one or more carbon-carbon triple bonds without aromatic bonds. The "alkynyl" as used herein includes an acyclic alkynyl group such as a linear or branched alkynyl group, and includes a cycloalkynyl group such as monocycloalkynyl, spirocycloalkynyl, fused cycloalkynyl, or bridged alkynyl. The alkynyl group can optionally contain one or more carbon-carbon double bonds. The alkynyl group may be unsubstituted or substituted.

As used herein, "alkynyl" includes an optionally substituted acyclic alkynyl group, preferably having from 2 to 20 carbon atoms, more preferably from 2 to 12 carbon atoms, most preferably from 2 to 6 carbon atoms; for example, acetynyl, propynyl, 1-butynyl, 2-butynyl, isobutynyl, 1-pentynyl, 2-pentynyl, isopenynyl, 3-methyl-3-butynyl , 1-hexynyl, 2-hexynyl, 3-hexynyl, 3-heptynyl, 1-octynyl, 1-nonynyl, 1-decynyl, 1-undecynyl , 1-dodecynyl and the like.

The "alkynyl" as used herein also includes optionally substituted cycloalkynyl (e.g., C8-C18 cycloalkynyl), for example, cyclooctynyl, and the like.

The "alkynyl" as used herein is optionally substituted by one or more (e.g., 1-3) substituents, wherein the choice and preference of the substituents are the same as those for the "alkyl".

The term "heterocyclyl" refers to a group derived from a monocyclic or polycyclic compound that is saturated or contains a carbon-carbon double bond or a carbon-carbon triple bond; which group contains 3 to 20 ring members (preferably 3 to 12 ring members, more preferably 3 to 8 ring members), wherein one or more ring members are selected from heteroatoms, and the remaining ring members are carbon; and any one of the rings has no aromaticity. The "heterocyclyl" as used herein also includes spiroheterocyclyl, fused heterocyclyl and bridged heterocyclyl. The heterocyclyl may be unsubstituted or substituted. The heterocyclyl group may be a heterocycloalkyl group, a heterocycloalkenyl group or a heterocycloalkynyl group. Examples of suitable monocyclic heterocyclcyl include, but are not limited to, piperidinyl, pyrrolidinyl, piperazinyl, azetidinyl, azacyclopropyl, morpholinyl, thietanyl, oxacyclopentyl (tetrahydrofuranyl), oxacyclohexyl (tetrahydropyranyl) and the like.

It will be understood that the "heterocyclyl" as used herein is optionally substituted by one or more (e.g., one to three) substituents, wherein the choice and preference of the substituents are the same as those for the "alkyl".

The term "aryl" refers to a group having a conjugated pi-electron system derived from 6 to 14 membered pure carbon monocyclic or fused polycyclic compound. The aryl ring may be fused to a heteroaromatic ring, a heterocyclic ring, cycloalkane, spirocycloalkane, fused cycloalkane, bridged cycloalkane, cycloalkenylene, spirocycloalkene, fused cycloalkene, bridged cycloalkene, cycloalkyne, spirocycloalkyne, fused cycloalkyne or bridged cycloalkyne. The aryl group may be unsubstituted or substituted. Examples thereof include, but are not limited to, phenyl, naphthyl, 2-methylphenyl, 3-methylphenyl, 4-methylphenyl, methoxyphenyl (such as 2-methoxyphenyl, 3 -methoxyphenyl, 4-methoxyphenyl), chlorophenyl (such as 2-chlorophenyl, 3-chlorophenyl, 4-chlorophenyl), fluorophenyl (such as 2-fluorophenyl, 3-fluorophenyl, 4-fluorophenyl), bromophenyl (such as 2-bromophenyl, 3-bromophenyl, 4-bromophenyl), 2-chloro-3-methylphenyl, 2-chloro-4-methylphenyl, 2-chloro-5-methylphenyl, 3-chloro-2-methylphenyl, 3-chloro-4-methylphenyl, 4-chloro-2-methyl phenyl, 4-chloro-3-methylphenyl, 5-chloro-2-methylphenyl, 2,3-dichlorophenyl, 2,5-dichlorophenyl, 3,4-dichlorophenyl, 2,3-dimethylphenyl, 3,4-dimethylphenyl, and the like.

The "aryl" as used herein are optionally substituted with from 1 to 4 or from 1 to 3 substituents, wherein the choice and preference of the substituents are the same as those for the "alkyl".

The term "heteroaryl" refers to a group derived from an aromatic system containing from 5 to 18 ring members, preferably from 5 to 14 ring members, one or four ring members of which are heteroatoms selected from the group consisting of oxygen, nitrogen and sulfur. The heteroaryl ring may be fused to an aryl ring, a heterocyclic ring, cycloalkane, spirocycloalkane, fused cycloalkane, bridged cycloalkane, cycloalkenylene, spirocycloalkene, fused cycloalkene, bridged cycloalkene, cycloalkyne, spirocycloalkyne, fused cycloalkyne or bridged cycloalkyne. The "heteroaryl" may be unsubstituted or substituted. Examples of heteroaryl groups include, but are not limited to, thienyl, furanyl, pyrrolyl, pyridyl, pyrimidinyl, imidazolyl, pyrazinyl, oxazolyl, thiazolyl, benzothienyl, benzofuranyl, benzooxazolyl, benzimidazolyl, indenyl, quinolyl, isoquinolyl and quinazolinyl, and the like.

The "heteroaryl" as used herein are optionally substituted with from 1 to 4 or from 1 to 3 substituents, wherein the choice and preference of the substituents are the same as those for the "alkyl".

The term "acyl" as used herein refers to RC(=O)-, wherein R is C1-C18 (preferably C1-C12, more preferably C1-C6) alkyl. Examples of "acyl" include, but are not limited to, formyl, acetyl, benzoyl, nicotinyl, propionyl, isobutyryl, oxalyl, and the like.

The acyl group RC(=O)- as used herein is optionally substituted by one or more (e.g., 1-3) substituents, wherein the choice and preference of the substituents are the same as those for the "alkyl".

The term "form a ring" as used herein means forming a cyclic structure such as a cycloalkane ring, a cycloalkene ring, a cycloalkyne ring, an aromatic ring, a heterocycloalkane ring, a heterocycloalkene ring, a heterocycloalkyne ring, a heteroaryl ring or the like wherein the cyclic structure may be a monocyclic, bicyclic or polycyclic structure including its fused ring, bridged ring, and spiro ring structure. Particularly, the ring formed by the substituents R¹ and R² herein is preferably a 3- to 12-membered ring, particularly preferably a 3- to 12-membered cycloalkane ring, cycloalkene ring, heterocycloalkane ring, and heterocycloalkene ring, and most preferably a 3- to 8-membered cycloalkane ring, cycloalkene ring, heterocycloalkane ring, and heterocycloalkene ring, such as a cyclopropane ring, a cyclobutane ring, a cyclopentane ring, a cyclohexane ring, a cycloheptane ring, a tetrahydrofuran ring, a tetrahydropyran ring and the like. The ring structure is optionally substituted with one or more (e.g., 1-3) substituents, wherein the choice and preference of the substituents are the same as those for the "alkyl".

Herein, a numerical range relating to the number of substituents, the number of carbon atoms, and the number of ring members represents an enumeration of all integers in the range, and the range is only a simplified representation thereof. For example:
"1-4 substituents" means 1, 2, 3 or 4 substituents;
"1-3 substituents" means a 1, 2 or 3 substituent;
"3 to 12-membered ring" means a 3, 4, 5, 6, 7, 8, 9, 10, 11 or 12-membered ring;
"3 to 8 membered ring" means a 3, 4, 5, 6, 7, or 8 membered ring;
"1-12 carbon atoms" or "C1-C12" means 1 (C1), 2 (C2), 3 (C3), 4 (C4), 5 (C5), 6 (C6) , 7 (C7), 8 (C8), 9 (C9), 10 (C10), 11 (C11) or 12 (C12) carbon atoms;
"1-6 carbon atoms" or "C1-C6" means 1 (C1), 2 (C2), 3 (C3), 4 (C4), 5 (C5) or 6 (C6) carbon atoms;
"2-6 carbon atoms" or "C2-C6" means 2 (C2), 3 (C3), 4 (C4), 5 (C5) or 6 (C6)carbon atoms;
"C3-C8" means 3 (C3), 4 (C4), 5 (C5), 6 (C6), 7 (C7) or 8 (C8) carbon atoms;
"3 to 8 ring members" means 3, 4, 5, 6, 7, or 8 ring members.

Thus, a numerical range associated with the number of substituents, the number of carbon atoms, and the number of ring members also encompasses any one of its subranges, and each subrange is also considered to be disclosed herein.

In some particularly preferred embodiments of the present application, the hypoxia-inducible factor-prolyl hydroxylase inhibitor (HIF-PHI) is the compound (6'-hydroxy-8'-oxo-3'-phenyl-8'H-spiro[cyclopentane-1,5'-indolizine]-7'-carbonyl)glycine disclosed in WO2018205928, or a pharmaceutically acceptable salt thereof. The compound (6'-hydroxy-8'-oxo-3'-phenyl-8'H-spiro[cyclopentane-1,5'-indolizine]-7'-carbonyl)glycine is the compound of Formula I wherein R₁ and R₂ together form a cyclopentane ring (i.e., - CH₂CH₂CH₂CH₂-), R₃ is phenyl, R₄ and R₅ are hydrogen, R₆ and R₆' are hydrogen, R₇ is hydrogen, R₈ is hydrogen, and X is an oxygen atom.

### II. Pharmaceutical Uses or Treatment Methods

A first aspect of the present application provides use of a hypoxia-inducible factor-prolyl hydroxylase inhibitor (HIF-PHI) in the manufacture of a medicament for treating a rare anemia.

A second aspect of the present application provides use of a hypoxia-inducible factor-prolyl hydroxylase inhibitor (HIF-PHI) in the manufacture of a medicament for increasing red blood cell count, hemoglobin levels, and/or hematocrit in a rare anemia.

A third aspect of the present application provides a method for treating a rare anemia, comprising administering to a patient in need thereof a therapeutically effective amount of a hypoxia-inducible factor-prolyl hydroxylase inhibitor (HIF-PHI).

A fourth aspect of the present application provides a method for increasing red blood cell count, hemoglobin levels, and/or hematocrit in a rare anemia, comprising administering to a patient in need thereof a therapeutically effective amount of a hypoxia-inducible factor-prolyl hydroxylase inhibitor (HIF-PHI).

A fifth aspect of the present application provides a hypoxia-inducible factor-prolyl hydroxylase inhibitor (HIF-PHI) for use in treating a rare anemia.

A sixth aspect of the present application provides use of a hypoxia-inducible factor-prolyl hydroxylase inhibitor (HIF-PHI) for treating a rare anemia.

As used herein, the term "patient" means all mammals and includes humans. Examples of patients include humans, cows, dogs, cats, goats, sheep, mice, pigs, rabbits, rats, or mice.

As used herein, the rare anemia can be selected from myelodysplastic syndrome-associated anemia (MDS anemia), β-thalassemia, or sickle cell disease (SCD anemia).

The hypoxia-inducible factor-prolyl hydroxylase inhibitors (HIF-PHIs) of the present application can also be used to treat complications of anemia or ischemic diseases, such as ischemic cerebrovascular disease, ischemic renal disease (IRD), ischemic cardiomyopathy (ICM), and the like.

When using the hypoxia-inducible factor prolyl hydroxylase inhibitors (HIF-PHIs) of the present application to treat rare anemias, it is generally necessary to administer a therapeutically effective amount of the active ingredient (i.e., the HIF-PHI) to the patient. As used herein, the term "therapeutically effective amount" refers to an amount of the active ingredient mentioned in the present application that, when administered to a patient, is effective to delay or eliminate the patient's symptoms or improve the patient's health condition. The specific dosage applied can be determined by a physician based on the patient's specific circumstances. The precise dosage to be employed will depend not only on the route of administration, the condition, the severity of the condition to be treated, and various physical factors related to the individual being treated, but may also be determined based on the judgment of the healthcare practitioner. In vitro or in vivo assays may optionally be used to help determine optimal dosage ranges.

For example, a compound of general Formula I of the present application can be administered to a patient at a daily dose of about 0.01-4000 mg, or 0.05-2000 mg, or 0.1-1000 mg, or 0.1-500 mg, 5-500 mg, 5-300 mg, 5-200 mg, 5-100 mg, 10-30 mg, 5-15 mg, 5-10 mg, 10-15 mg, or 30-50 mg. However, the specific dosage used may vary. Those skilled in the art know how to determine the optimal dosage for a particular patient.

For therapeutic applications, HIF-PHIs are usually administered to a patient in the form of a pharmaceutical composition comprising at least one above-mentioned compound as an active ingredient, optionally together with a pharmaceutically acceptable adjuvant and/or excipient, and a pharmaceutically acceptable solid or liquid carrier.

The pharmaceutical composition of the present application can be formulated into various pharmaceutical dosage forms suitable for oral administration as needed. Thus, if a solid carrier is used, the preparation can be in the form of a tablet, a hard gelatin capsule where the active ingredient is present in powder or granular form, or a troche or lozenge. Solid carriers can include conventional excipients such as binders, fillers, tableting lubricants, disintegrants, wetting agents, and the like. The tablets can be film-coated by conventional techniques if desired. If a liquid carrier is used, the preparation can be in the form of a syrup, emulsion, soft gelatin capsule, aqueous or non-aqueous liquid suspension, or can be a dry product for reconstitution with water or other suitable vehicle before use. Liquid preparations may contain conventional additives such as suspending agents, emulsifying agents, wetting agents, non-aqueous vehicles (including edible oils), preservatives, and flavoring and/or coloring agents.

These pharmaceutical compositions (or pharmaceutical preparations) may also contain various excipients, for example, preservatives, wetting agents, emulsifying agents, and dispersing agents. Inhibition of action of microorganisms can be ensured by various antibacterial and antifungal agents, for example, parabens, chlorobutanol, phenol, sorbic acid, and the like. It may also be desirable to include isotonic agents, for example, sugars, sodium chloride, and the like. Prolonged absorption of an injectable pharmaceutical form can be brought about by use of agents delaying absorption, for example, aluminum monostearate and gelatin.

Solid dosage forms for oral administration include capsules, tablets, pills, powders, and granules. In such solid dosage forms, the active compound is mixed with at least one inert excipient (or carrier), such as sodium citrate or dicalcium phosphate, and/or with (a) fillers or extenders such as starches, lactose, sucrose, glucose, mannitol, and silicic acid; (b) binders such as carboxymethylcellulose, alginates, gelatin, polyvinylpyrrolidone, sucrose, and acacia; (c) humectants such as glycerol; (d) disintegrating agents such as agar-agar, calcium carbonate, potato or tapioca starch, alginic acid, certain complex silicates, and sodium carbonate; (e) solution retarders such as paraffin; (f) absorption accelerators such as quaternary ammonium compounds; (g) wetting agents such as cetyl alcohol and glycerol monostearate; (h) adsorbents such as kaolin and bentonite; and (i) lubricants such as talc, calcium stearate, magnesium stearate, solid polyethylene glycols, sodium lauryl sulfate, or mixtures thereof.

In soft and hard-filled gelatin capsules, similar types of solid pharmaceutical compositions (or pharmaceutical preparations) may also be employed as fillers using excipients such as lactose and high molecular weight polyethylene glycols, and the like.

Solid dosage forms such as tablets, dragees, capsules, pills, and granules can be prepared with coatings and shells such as enteric coatings and others well known in the art. They may contain opacifying agents, and may also be of such composition that they release the active compound or compounds in a delayed manner in a certain part of the intestinal tract. The active components can also be in micro-encapsulated form, if appropriate, with one or more of the above-mentioned excipients.

Liquid dosage forms for oral administration include pharmaceutically acceptable emulsions, solutions, dispersions, syrups, and elixirs. In addition to the active compounds, liquid dosage forms may contain inert diluents commonly used in the art, such as water or other solvents, solubilizing agents and emulsifiers such as ethanol, isopropanol, ethyl carbonate, ethyl acetate, benzyl alcohol, benzyl benzoate, propylene glycol, 1,3-butylene glycol, and dimethylformamide, oils such as cottonseed oil, groundnut oil, corn oil, olive oil, castor oil, and sesame oil, glycerol, tetrahydrofurfuryl alcohol, polyethylene glycols, and fatty acid esters of sorbitan, or mixtures of these substances, and the like.

Besides such inert diluents, the pharmaceutical compositions (or pharmaceutical preparations) can also include adjuvants such as wetting agents, emulsifying and suspending agents, sweetening, flavoring, and perfuming agents.

In addition to the active compounds, suspensions may contain suspending agents such as ethoxylated isostearyl alcohols, polyoxyethylene sorbitol, sorbitan esters, microcrystalline cellulose, aluminum metahydroxide, bentonite, agar-agar, and tragacanth, or mixtures of these substances, and the like.

The amount of the HIF-PHI compound in the pharmaceutical composition (or pharmaceutical preparation) can be appropriately determined by a person skilled in the art as needed.

In some embodiments, the HIF-PHI or a pharmaceutically acceptable salt thereof is present in each unit pharmaceutical preparation in an amount of: about 1-1000 mg, about 1-10 mg, about 10 mg, about 5 mg, about 4.5 mg, about 1-3 mg, about 2-4 mg, about 3-5 mg, about 4-6 mg, about 5-7 mg, about 6-8 mg, about 7-9 mg, about 8-10 mg, about 9-11 mg, about 10-12 mg, about 4.5-5 mg, 20 mg, 30 mg, 30-100 mg, about 40 mg, about 50 mg, about 60 mg, about 70 mg, about 80 mg, about 90 mg, about 10-30 mg, about 30-50 mg, about 50-70 mg, about 10-90 mg, about 10-800 mg, about 10-700 mg, about 10-600 mg, about 10-500 mg, about 10-400 mg, about 10-300 mg, or about 10-200 mg, or in an amount within any range defined by any of these values.

In some embodiments, the HIF-PHI or a pharmaceutically acceptable salt thereof is present in each unit pharmaceutical preparation in an amount of: about 0.01-0.2 mg, about 0.2-0.4 mg, about 0.4-0.6 mg, about 0.6-0.8 mg, about 0.8-1 mg, about 1-1.2 mg, about 1.2-1.4 mg, about 1.4-1.6 mg, about 1.6-1.8 mg, about 1.8-2 mg, about 2-2.2 mg, about 2.2-2.4 mg, about 2.4-2.6 mg, about 2.6-2.8 mg, about 2.8-3 mg, about 3-3.2 mg, about 3.2-3.4 mg, about 3.4-3.6 mg, about 3.6-3.8 mg, about 3.8-4 mg, about 3.9-4.1 mg, about 4-4.2 mg, about 0.2-0.4 mg, about 0.2-0.6 mg, about 0.2-0.8 mg, about 0.2-1 mg, about 0.2-1.2 mg, about 0.2-1.4 mg, about 0.2-1.6 mg, about 0.2-1.8 mg, about 0.2-2.0 mg, 0.2-2.5 mg, about 0.2-3.0 mg, about 0.2-3.5 mg, about 0.2-4.0 mg, about 5-10 mg, about 10-15 mg, about 15-20 mg, about 20-25 mg, about 25-30 mg, about 30-40 mg, about 40-50 mg, about 50-60 mg, about 60-70 mg, about 70-80 mg, about 80-90 mg, about 90-100 mg, about 100-120 mg, about 120-140 mg, about 140-150 mg, about 150-160 mg, about 160-180 mg, about 180-200 mg, about 200-220 mg, about 220-240 mg, about 10-500 mg, about 50-400 mg, about 50-300 mg, about 100-250 mg, about 1-10 mg, about 10-200 mg, about 10-150 mg, about 10-100 mg, about 10-180 mg, about 10-160 mg, about 10-140 mg, about 10-120 mg, about 10-100 mg, about 10-20 mg, about 20-30 mg, about 30-40 mg, about 40-50 mg, about 50-60 mg, about 60-70 mg, about 70-80 mg, about 80-90 mg, about 90-100 mg, about 100-120 mg, about 120-140 mg, about 140-160 mg, about 160-180 mg, about 180-200 mg, about 200-220 mg, about 220-240 mg, about 240-250 mg, about 250-260 mg, about 260-280 mg, about 280-300 mg, about 300-350 mg, about 350-400 mg, about 25 mg, about 50 mg, about 100 mg, about 250 mg, or in an amount within any range defined by any of these values.

In some embodiments, the HIF-PHI or a pharmaceutically acceptable salt thereof is present in each unit pharmaceutical preparation in a percentage relative to the total weight of the pharmaceutical preparation of: about 0.1% (w/w) to about 10% (w/w), about 0.05% (w/w) to about 5% (w/w), about 0.2% (w/w) to about 15% (w/w), about 0.5% (w/w) to about 30% (w/w), about 1% (w/w) to about 25% (w/w), about 2% (w/w), about 3% (w/w), about 4% (w/w), about 5% (w/w), at least about 10% (w/w), at least about 20% (w/w), at least about 50% (w/w), at least about 70% (w/w), at least about 80% (w/w), about 10% (w/w) to about 30% (w/w), about 10% (w/w) to about 20% (w/w), about 20% (w/w) to about 30% (w/w), about 30% (w/w) to about 50% (w/w), about 30% (w/w) to about 40% (w/w), about 40% (w/w) to about 50% (w/w), about 50% (w/w) to about 80% (w/w), about 50% (w/w) to about 60% (w/w), about 70% (w/w) to about 80% (w/w), or about 80% (w/w) to about 90% (w/w), or in a percentage within any range defined by any of these values.

The preparation of the pharmaceutical compositions (or pharmaceutical preparations) can employ methods well-known or commonly used in the art, which generally involve the step of mixing the active ingredient HIF-PHI with pharmaceutically acceptable carriers, adjuvants, or excipients, and optional post-treatment or processing steps (e.g., drying, granulation, encapsulation, etc.).

### Examples

The examples described below are part of the embodiments of the present invention, not all of the embodiments. The detailed description of the embodiments of the present invention is not intended to limit the scope of the claimed invention, but merely represents selected embodiments of the present invention. Based on the embodiments of the present invention, all other embodiments obtained by a person of ordinary skill in the art without departing from the principles of the present invention and without creative effort shall fall within the protection scope of the present invention.

For the sake of brevity, some materials, equipment, and method steps conventionally used in the art are not individually specified in the examples. All process methods and analytical testing procedures (and related parameters) not specifically noted in the examples are carried out according to those commonly used by those skilled in the art; and materials, reagents, and equipment not specified with a specific source are conventional laboratory materials and equipment, which can be obtained through commercial channels.

### Example 1

### Synthesis of (6'-hydroxy-8'-oxo-3'-phenyl-8'H-spiro[cyclopentane-1,5'-indolizine]-7'-carbonyl)glycine (1)

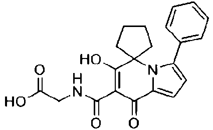

Compound 1 was synthesized according to the method of Example 24 in WO2018205928.

The structure of the compound was confirmed by liquid chromatography-mass spectrometry (LCMS) or nuclear magnetic resonance (NMR). NMR chemical shifts (δ) are expressed in units of parts per million (ppm). NMR was measured using a Bruker-500 type NMR spectrometer, with deuterated dimethyl sulfoxide (dmso-d6), deuterated chloroform (CDCl₃), etc., as a measurement solvent, and tetramethylsilane (TMS) as an internal standard. LCMS was measured using a Shimadzu LCMS-2020 or Thermo UltiMate 3000.

Thin-layer chromatography silica gel plates used were Yantai Huanghai HSGF254 or Yantai Qingdao GF254 silica gel plates from Shandong Province. Column chromatography generally used Yantai Huanghai silica gel (200-300 mesh) from Shandong Province as a carrier.

All starting materials used in this example were purchased from chemical suppliers or could be synthesized by literature methods.

Abbreviations possibly used in this example are as follows:
DMSO-d6: Dimethyl sulfoxide where all six hydrogen atoms are replaced by deuterium
CDCl₃: Deuterated chloroform
CAS: Chemical Abstracts Service registration number
NMR: Nuclear magnetic resonance
LCMS: Liquid chromatography-mass spectrometry
ESI: Electrospray ionization
ppm: Parts per million
δ: NMR chemical shift
TMS: Tetramethylsilane
s: NMR singlet
d: NMR doublet
t: NMR triplet
br: NMR broad peak
CDI: Carbonyldiimidazole
DCC: N,N'-Dicyclohexylcarbodiimide
NBS: N-Bromosuccinimide

The main synthetic steps for Compound 1 were as follows.

### Step 1

### 1-(2-Phenyl-1H-pyrrol-1-yl)cyclopentane-1-carboxylic acid (1a)

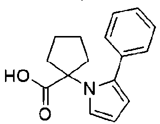

3-(1,3-dioxan-2-yl)-1-phenylpropan-1-one (prepared according to the method described in patent document WO/2011/042477) (500 mg) and 1-amino-cyclopentane-1-carboxylic acid (421 mg) were refluxed in 8 mL of acetic acid for 16 hours. Then, after cooling the reaction mixture, acetic acid was evaporated as much as possible; and then water and ethyl acetate were added for dilution. The resulting organic phase was collected, and the organic phase was washed several times with dilute aqueous sodium chloride solution until the aqueous phase was nearly neutral; then the ethyl acetate layer was dried over anhydrous sodium sulfate, filtered, concentrated, and purified by column chromatography to obtain Compound 1a. LCMS ESI(+): 256 (M+1)⁺. ¹H NMR (500 MHz, DMSO-d6) δ (ppm): 12.95 (br, s, 1H), 7.35-7.33 (m, 3H), 7.25-7.22 (m, 2H), 7.00-6.99 (m, 1H), 6.04 (m, 1H), 5.95 (m, 1H), 2.14-2.11 (m, 2H), 2.01-1.97 (m, 2H), 1.60-1.57 (m, 4H).

### Step 2

### Dimethyl 2-(1-(2-phenyl-1H-pyrrol-1-yl)cyclopentane-1-carbonyl)malonate (1b)

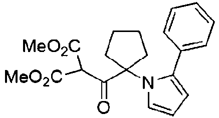

Following the synthetic route for the second step of Example 15 (compound 15b) in WO2018205928, the starting material 15a was replaced with 1a to yield 1b. LCMS ESI(+): 370 (M+1)⁺. ¹H NMR (500 MHz, DMSO-d6) δ 7.28 - 7.22 (m, 3H), 7.20 - 7.15 (m, 2H), 7.11 (dd, J = 3.2, 1.8 Hz, 1H), 6.15 (t, J = 3.3 Hz, 1H), 5.93 (dd, J = 3.5, 1.7 Hz, 1H), 5.13 (s, 1H), 3.56 (s, 6H), 2.06 (d, J = 8.0 Hz, 2H), 1.59 (t, J = 12.2 Hz, 2H), 1.33 (s, 2H), 1.16 (s, 2H).

### Step 3

### Methyl 6'-hydroxy-8'-oxo-3'-phenyl-8'H-spiro[cyclopentane-1,5'-indolizine]-7'-carboxylate (1c)

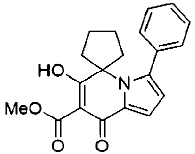

Following the synthetic route for the third step of Example 15 (compound 15c) in WO2018205928, the starting material 15b was replaced with 1b to yield 1c. LCMS ESI(+): 338 (M+1)⁺. ¹H NMR (500 MHz, DMSO-d6) δ 13.84 (s, 1H), 7.54-7.43 (m, 5H), 7.09 (d, J = 4.0 Hz, 1H), 6.35 (d, J = 4.0 Hz, 1H), 3.81 (s, 3H), 2.30-2.21 (m, 2H), 2.10-2.01 (m, 2H), 1.48-1.38 (m, 2H), 1.00-0.89 (m, 2H).

### Step 4

### (6'-Hydroxy-8'-oxo-3'-phenyl-8'H-spiro[cyclopentane-1,5'-indolizine]-7'-carbonyl)glycine (1)

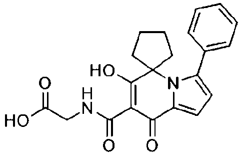

Following the synthetic route for the fourth step of Example 15 (compound 15) in WO2018205928, the starting material 15c was replaced with 1c to yield Compound 1. LCMS ESI(+): 381 (M+1)⁺. ¹H NMR (500 MHz, DMSO-d6) δ (ppm) 17.96 (s, 1H), 12.97 (s, 1H), 9.89-9.87 (d, J = 5.5 Hz, 1H), 7.53-7.46 (m, 5H), 7.08-7.07 (d, 1H), 6.37-6.36 (d, 1H), 4.08-4.07 (d, J = 5.5 Hz, 2H), 2.35-2.20 (m, 2H), 2.18-2.10 (m, 2H), 1.60-1.46 (m, 2H), 0.98-0.85 (m, 2H).

### Example 2

### Inhibitory Effect of Compound on HIF PHD-2

In this example and the following examples, Compound 1 prepared in Example 1 was used for testing, and it is referred to as "Comp-1".

Stock solutions of the following reagents were prepared: HEPES buffer (pH 7.4, 50 mM), HIF-1α peptide (sequence: DLDLEMLAPYIPMDDDFQL; Genscript) solution in DMSO/HEPES (0.5 mM; preparation method: first prepare a 20 mM solution of this peptide in DMSO, then dilute it 40-fold with the HEPES buffer), ascorbic acid solution (10 mM), FeSO₄ in hydrochloric acid solution (0.1 mM FeSO₄, 10 µM HCl), 2-oxoglutaric acid solution (2-OG: 1 mM), EDTA solution (pH 8.0; 0.5 M), and eight concentrations of Comp-1/DMSO solutions (concentrations: 4000 µM, 1333 µM, 444.4 µM, 148.1 µM, 49.4 µM, 16.5 µM, 5.5 µM, and 1.82 µM). The following reagents were added to nine Eppendorf tubes: 50 µL HEPES solution, 10 µL ascorbic acid solution, 10 µL FeSO₄ in HCl solution, 10 µL 2-OG solution, 10 µL HIF-1α peptide solution; then 5 µL of each of the eight Comp-1/DMSO solutions and 5 µL of DMSO were added separately; finally, 5 µL of PHD2 enzyme (Active Motif; concentration: 0.2 µg/µL) was added to each tube. Each of the resulting reaction systems was incubated at 30 °C in dark for 2 hours. Finally, 10 µL of EDTA solution was added to each tube to terminate the reaction. The liquid from each reaction system was filtered through a 0.45 µm filter, and then HIF-peptide and hydroxylated HIF-peptide were separated by high-performance liquid chromatography. The concentrations of HIF-peptide and hydroxylated peptide were quantified using a UV absorption detector to calculate their ratio. The IC₅₀ was calculated using GraphPad Prism 6.0 software. IC₅₀ of Comp-1 was 2.36 µM against HIF PHD-2.

### Example 3

### Effect of HIF-PH Inhibitor and EPO on MDS Model Mice

Throughout the experimental stage, animals were housed in cages in an animal room with 24-hour continuous central air conditioning, temperature set at 20-26°C, relative air humidity set at 40-70%, and a 12-hour light/dark cycle. All animals had free access to food and sterilized drinking water. The C57 mice used in the experiment contained three knocked-in genes in their genome: Vav1-Cre, LSL, and c-Myc(T58A/S62E). Only animals positive for all three genes were used in the experiment. Expression of Vav1-Cre can induce cleavage at the LSL site, thereby allowing tTA gene expression. After induction with Doxycycline, tTA can bind to promoter of the c-Myc(T58A/S62E) gene, driving its expression, so that the animals develop an MDS phenotype. When the mice reached approximately 8 weeks of age, modeling commenced. They were fed a special diet (containing 200 mg doxycycline per kg of feed) for 4 weeks. Mice were anesthetized on days 0, 14, and 28, and 70 µl of blood was collected from the orbit to measure RBC, Hb, and HCT levels, confirming successful establishment of the MDS model. Subsequently, the mice were randomly divided into 3 groups, with 10 mice per group: Vehicle control group (0.1 M meglumine), Comp-1 low-dose group (10 mg/kg), and Comp-1 high-dose group (30 mg/kg). The dosing or vehicle volume was 6.67 mL/kg. The dosing or vehicle frequency for each group was oral gavage three times per week (TIW) for 4 consecutive weeks. That is, from week 4 to week 8 constituted the treatment period. Mice were anesthetized 14 days (experimental week 6) and 28 days (experimental week 8) after the start of dosing, and 70 µl of blood was collected from the orbit. Collected blood samples were placed in heparin-anticoagulant tubes, and RBC, Hb, and HCT levels in the blood were measured. After blood collection at the final time point for each animal, the animals were euthanized, and corresponding organ tissues were collected for storage and potential future use. Detection instrument: Element HT5 Veterinary Hematology Analyzer; Supplier: HESKA. Data analysis was performed using GraphPad Prism 9.0.

### Experimental Results:

(1) Effect on RBC: After oral gavage of Comp-1, RBC levels in the blood increased significantly. After 2 weeks of dosing, RBC values in the 30 mg/kg and 10 mg/kg groups were significantly higher than those in the vehicle group (7.11 M/µL vs 6.33 M/µL vs 5.57 M/µL). After 4 weeks of dosing, RBC in the treatment groups continued to rise (8.46 M/µL vs 7.17 M/µL vs 5.27 M/µL), and RBC in the 30 mg/kg group recovered to near premodeling levels (8.46 M/µL vs 9.01 M/µL). The experimental results were summarized in table below:

| Groups | doses (mg/kg) | RBC Red Blood Cell Count (M/µL): M stands for 10⁶ | | | |
|---|---|---|---|---|---|
| | | Pre- modeling Week 0 | Before dosing Week 4 | After 2 weeks of dosing Week 6 | After 4 weeks of dosing Week 8 |
| Vehicle | 0.1 M Meglumine | 9.02 | 6.01 | 5.57 | 5.27 |
| Comp-1 | 10 | 9.00 | 6.03 | 6.33 | 7.17 |
| Comp-1 | 30 | 9.01 | 5.90 | 7.11 | 8.46 |

(2) Effect on Hb: After oral gavage of Comp-1, Hb levels in MDS mice increased significantly. After 2 weeks of dosing, Hb values in the 30 mg/kg and 10 mg/kg groups were significantly higher than those in the vehicle group (13.56 g/dL vs 10.97 g/dL vs 8.72 g/dL). After 4 weeks of dosing, Hb levels in the treatment groups continued to rise (14.91 g/dL vs 11.77 g/dL vs 8.19 g/dL), and Hb level in the 30 mg/kg group recovered to premodeling levels (14.91 g/dL vs 14.37 g/dL). The experimental results were summarized in table below:

| Groups | Doses (mg/kg) | Hb Hemoglobin content (g/dL) | | | |
|---|---|---|---|---|---|
| | | Premodeling Week 0 | Before dosing Week 4 | After 2 weeks of dosing Week 6 | After 4 weeks of dosing Week 8 |
| Vehicle | 0.1 M Meglumine | 14.45 | 9.67 | 8.72 | 8.19 |
| Comp-1 | 10 | 14.90 | 9.52 | 10.97 | 11.77 |
| Comp-1 | 30 | 14.37 | 9.54 | 13.56 | 14.91 |

(3) Effect on HCT: After oral gavage of Comp-1, HCT levels in MDS mice increased significantly. After 2 weeks of dosing, HCT values in the 30 mg/kg and 10 mg/kg groups were significantly higher than those in the vehicle group (43.13 % vs 34.36 % vs 27.69 %). After 4 weeks of dosing, HCTs in the treatment groups continued to rise (47.41 % vs 37.52 % vs 27.13 %), and HCT in the 30 mg/kg group recovered to slightly above premodeling levels (47.41 % vs 43.32 %). The experimental results were summarized in table below:

| Groups | Doses (mg/kg) | HCT Hematocrit (%) | | | |
|---|---|---|---|---|---|
| | | Premodeling Week 0 | Before dosing Week 4 | After 2 weeks of dosing Week 6 | After 4 weeks of dosing Week 8 |
| Vehicle | 0.1 M Meglumine | 43.08 | 27.47 | 27.69 | 27.13 |
| Comp-1 | 10 | 43.35 | 28.12 | 34.36 | 37.52 |
| Comp-1 | 30 | 43.32 | 28.01 | 43.13 | 47.41 |

Thus, the test substance Comp-1 at 10 and 30 mg/kg was able to effectively increase RBC, Hb, and HCT values in the blood of MDS mice, significantly ameliorating the anemia symptoms in MDS mice.

As a control, the same experiment was repeated using Roxadustat (30 mpk, oral gavage, TIW) and EPO (50 U/30 g). Neither Roxadustat nor EPO improved RBC, Hb, or HCT values in the MDS mouse model.

### Example 4

### Effect of Comp-1 on Homozygous β-Thalassemia Mice

Throughout the experimental stage, animals were housed in cages in an animal room with 24-hour continuous central air conditioning, temperature set at 20-26°C, relative air humidity set at 40-70%, and a 12-hour light/dark cycle. All animals had free access to food (irradiated sterile feed) and water (prepared by an ultrapure water system). The mouse strain used in the experiment was: Hbb^{d3th}/^{d3th} mice; female and male, 9-15 weeks old, weighing 25-30 grams. The breeding method involved one male paired with two females. Male mice with the Hbb^{d3th}/^{d3th} genotype, and female mice with the Hbb^{d3th}/+ genotype were selected. At one week of age, tissue DNA was extracted from toe clips of offspring. Subsequently, PCR amplification and agarose gel electrophoresis for band separation were used for genotyping. Before the experiment, secondary genotyping was performed on mice of appropriate age, and mice with no significant differences in body weight and appearance were selected as experimental animals. Vehicle 1 (0.1 M meglumine aqueous solution) was prepared by dissolving an appropriate amount of meglumine (0.586 g) in an appropriate amount of deionized water (30 mL) with stirring to prepare the vehicle. After preparation, the vehicle was aliquoted into daily doses and stored at 5±3 °C. Before administration, it was stirred at room temperature for at least 10 minutes and stirred continuously during the dosing process.

Comp-1: The required amount of Comp-1 was weighed, completely dissolved in an appropriate volume of Vehicle 1, with stirring and sonicating to form a solution. After visually observing dissolution, vehicle was gradually added to reach the specified volume. Before daily dosing, the formulations (including vehicle) were removed from refrigerator and allowed to reach room temperature. Formulations were stirred at room temperature for at least 10 minutes before administration and stirred throughout the dosing process. Information on animal grouping, doses, experiment duration, etc., was shown in the table below. The route of administration was oral gavage for all groups, with a dosing volume of 10 mL/kg for all groups, and a dosing frequency of daily for a total of 42 days.

| Group | Animal Number | Animal Sex | Drug | Dosing Time | Dose, mg/kg |
|---|---|---|---|---|---|
| G1 | 2+3 | Female + Male | Vehicle 1 | Days 1-42 | 0 |
| G2 | 2+3 | Female + Male | Comp-1 | Days 1-42 | 5 |
| G3 | 2+3 | Female + Male | Comp-1 | Days 1-42 | 10 |
| G4 | 2+3 | Female + Male | Comp-1 | Days 1-42 | 15 |

### CBC Analysis

On days 0, 15, 29, and 43 of dosing, 50 µl of blood was collected from orbit of mice, stored at 4°C, and allowed to return to room temperature before analysis. Samples were then gently mixed and added to pre-prepared diluent (blood: diluent = 1:6). Data were obtained using the instrument's pre-dilution mode for detection. Data statistics were processed and analyzed using Microsoft Office Excel (Microsoft Corporation, version 2013, Redmond, WA, USA) and GraphPad Prism 7.0. Red blood cell counts and percentages were reported with two significant figures. T-test analysis was used for comparisons of blood cell counts and percentages between groups and within groups at different time points.

### Experimental Results:

(1) Effect on RBC Count: RBC count gradually increased after dosing, with the 15 mpk dosing group reaching a peak on day 29, then stabilizing. Hematocrit and hemoglobin concentration stabilized after day 29, and the treatment groups were higher than the control group, showing a clear dose-response relationship. Compared to pre-dose levels, RBC in the 10 and 15 mpk groups gradually increased on D15, D29, and D43 in a dosedependent manner. Although the 5 mpk group showed no significant difference at various time points compared to pre-dose, RBC showed a slow upward trend. RBCs in the 10 and 15 mpk groups were significantly increased on D15, D29, and D43 compared to pre-dose, with the increase being more significant in the 15 mpk group. The experimental results were summarized in table below:

| RBC Red Blood Cell Count (10¹²/L) | | | | | |
|---|---|---|---|---|---|
| Groups | Doses | Day 0 | Day 15 | Day 29 | Day 43 |
| Vehicle | Meglumine | 7.83 | 8.05 | 8.27 | 8.29 |
| Comp-1 | 5 mpk | 7.90 | 8.35 | 8.59 | 8.58 |
| Comp-1 | 10 mpk | 7.90 | 8.60## | 9.15# | 9.30## |
| Comp-1 | 15 mpk | 7.77 | 8.95# | 9.72#### | 9.45#### |

(2) Effect on HGB Hemoglobin Content: HGB content increased in all treatment groups over time. Compared to the vehicle group, the 15 mpk group showed statistically significant differences at D29 and D43 (*P<0.05; ***P<0.001). HGB contents in the 10 and 15 mpk groups were significantly increased at D15, D29, and D43, showing a clear dose-dependency, and were statistically significant compared to their respective pre-dose levels. In each group, comparison with pre-dose and after dossing at different periods were summarized in table below: #, P<0.05; ##, P<0.01; ###, P<0.001; ####, P<0.0001.

| HGB Hemoglobin content (g/dL | | | | | |
|---|---|---|---|---|---|
| Groups | Doses | Day 0 | Day 15 | Day 29 | Day 43 |
| Vehicle | Meglumine | 76.00 | 80.40 | 78.00 | 78.00 |
| Comp-1 | 5 mpk | 74.60 | 81.20 | 83.00 | 80.20 |
| Comp-1 | 10 mpk | 74.80 | 83.60# | 88.04# | 89.40## |
| Comp-1 | 15 mpk | 75.00 | 90.17## | 97.5#### | 94.83#### |

(3) Effect on HCT Hematocrit: Comp-1 at 5, 10, and 15 mpk increased HCT percentage. HCT gradually increased over dosing time, showing a certain degree of dose-dependency. Compared to the vehicle group, all three dose groups showed statistical significance at the experimental endpoint D43 (low dose P<0.05, medium dose P<0.01, high dose P<0.01). The effect of the high-dose group in increasing HCT at D43 was significantly higher than that of the medium and low-dose groups (P<0.05, P<0.01). Compared to their respective pre-dose levels, HCT% was significantly increased in the low, medium, and high-dose groups after dosing, with a clear dose-dependency (#, P<0.05; ##, P<0.01; ###, P<0.001; ####, P<0.0001). The experimental results were summarized in table below:

| HCT Hematocrit (%) | | | | | |
|---|---|---|---|---|---|
| Groups | Doses | Day 0 | Day 15 | Day 29 | Day 43 |
| Vehicle | Meglumine | 27.78 | 31.12 | 29.05 | 28.48 |
| Comp-1 | 5 mpk | 27.58 | 31.16# | 32.10## | 31.04## |
| Comp-1 | 10 mpk | 26.20 | 31.92## | 33.02## | 33.36### |
| Comp-1 | 15 mpk | 27.48 | 34.50## | 36.13### | 36.10#### |

Thus, Comp-1 can significantly increase red blood cell count, hemoglobin content, and hematocrit in thalassemia mice, indicating that Comp-1 has a therapeutic effect on anemia in homozygous d3^{th} thalassemia genotype mice.

### Example 5

### Effect of HIF PH Inhibitors and EPO on SCD Anemia Mice

Throughout the experimental stage, animals were housed in cages in an animal room with 24-hour continuous central air conditioning, temperature set at 20-26°C, relative air humidity set at 40-70%, and a 12-hour light/dark cycle. All animals had free access to food and sterilized drinking water. The animal strain was B6.Cg-Hbb^{tm2(HBG1HBB)Tow}/Hbat^{m1(HBG1HBB)Tow}/ShmtJ mice, a Townes SCD model mouse where mouse endogenous hemoglobin is replaced by knocked-in human hemoglobin. Two weeks after birth, mice were ear-punched for identification, and the ear tissue was collected for PCR analysis to determine its genotype. Mice confirmed as B6.Cg-Hbb^{tm2(HBG1HBB)Tow}/Hbat^{m1(HBG1HBB)Tow}/ShmtJ were grouped. When mice were around 4 weeks old, blood was collected from its orbit to measure RBC, Hb, and HCT values to determine if the animal showed symptoms of anemia. Blood sampling was performed every other week. Mice showing symptoms of anemia were selected for dosing. Animals were randomly divided into groups, 6 animals per group: Vehicle control group (0.1 M meglumine aqueous solution; oral gavage; volume: 6.67 mL/kg), Comp-1 group (30 mpk; oral gavage; volume: 6.67 mL/kg), and EPO group (50 U/30 g body weight; SC injection; MyBioSource, cat. #: MBS650094, erythropoietin alpha). Dosing was administered three times a week (TIW) for 4 consecutive weeks. On days 14 and 28 of dosing, 70 µL of blood was collected from orbit. Collected blood samples were placed in heparin-anticoagulant tubes. 15 µL of the blood sample from the anticoagulant tube was aspirated and added to an Element HT5 Veterinary Hematology Analyzer. RBC, Hb, and HCT in the blood were measured according to the instrument's operating instructions. Data analysis was performed using GraphPad Prism 9.0.

### Experimental Results:

From the arithmetic mean values of the RBC count, hemoglobin content, and hematocrit of each group of mice before dosing shown in the three tables below, it can be seen that mice in all groups were severely anemic, indicating successful modeling.
(1) Effect on RBC Red Blood Cell Count: As shown in the table below, over time, RBC count in the vehicle control group continued to decline, indicating worsening anemia. Even in mice treated with the EPO positive control (which can be used for anemia like CKD), RBC count continued to decline over time, and the degree of anemia worsened. RBC counts in the EPO group and vehicle control group were similar at the same time points, indicating that EPO could not induce an increase in RBC count. In contrast, the Comp-1 group (30 mg/kg) showed a substantial increase in RBC count after 2 and 4 weeks of dosing compared to pre-dose (8.88 M/µL, 9.09 M/µL vs 6.34 M/µL).

| Groups | Doses | Arithmetic mean of RBC red blood cell count (M/µL): M stands for 10⁶ | | |
|---|---|---|---|---|
| | | Before dosing Week 0 | After 2 weeks of dosing Week 2 | After 4 weeks of dosing Week 4 |
| Vehicle | 0.1 M Meglumine | 6.33 | 5.69 | 5.42 |
| Comp-1 | 30 mg/kg | 6.34 | 8.88 | 9.09 |
| EPO | 50 U/30 g | 6.25 | 5.80 | 5.43 |

(2) Effect on HGB Hemoglobin Content: As shown in the table below, over time, hemoglobin content of mice in the vehicle control group continued to decline, and anemia worsened. Even in mice treated with the EPO positive control, hemoglobin content continued to decline over time, and anemia worsened. HGB levels in the EPO group and vehicle control group were similar at the same time points, indicating that EPO could not induce an increase in hemoglobin content. In contrast, the Comp-1 group (30 mg/kg) showed a substantial increase in hemoglobin content after 2 and 4 weeks of dosing compared to pre-dose (11.63 g/dL, 12.10 g/dL vs 8.47 g/dL), approaching normal levels.

| Groups | Doses | Arithmetic mean of HGB hemoglobin content (g/dL) | | |
|---|---|---|---|---|
| | | Before dosing Week 0 | After 2 weeks of dosing Week 2 | After 4 weeks of dosing Week 4 |
| Vehicle | 0.1 M Meglumine | 8.63 | 7.57 | 6.93 |
| Comp-1 | 30 mg/kg | 8.47 | 11.63 | 12.10 |
| EPO | 50 U/30 g | 8.37 | 7.52 | 6.87 |

(3) Effect on HCT Hematocri: As shown in the table below, over time, hematocrit levels of mice in the vehicle control group continued to decline, and anemia worsened. Even in mice treated with the EPO positive control, hematocrit levels continued to decline over time, and anemia worsened. HCT levels in the EPO group and vehicle control group were similar at the same time points, indicating that EPO could not increase hematocrit levels. In contrast, the Comp-1 group (30 mg/kg) showed a substantial increase in hematocrit levels after 2 and 4 weeks of dosing compared to pre-dose (40.05%, 41.45% vs 27.20%), approaching normal levels.

| Groups | Doses | Arithmetic mean of HCT hematocrit (%) | | |
|---|---|---|---|---|
| | | Before dosing Week 0 | After 2 weeks of dosing Week 2 | After 4 weeks of dosing Week 4 |
| Vehicle | 0.1 M Meglumine | 26.72 | 24.97 | 23.47 |
| Comp-1 | 30 mg/kg | 27.20 | 40.05 | 41.45 |
| EPO | 50 U/30 g | 27.00 | 25.02 | 23.25 |

As controls, the same experiment was repeated using Roxadustat (30 mpk, 60 mpk, oral gavage, TIW) and Daprodustat (30 mpk, oral gavage, TIW).

At the end of the four-week experiment, neither dose of Roxadustat improved RBC, Hb, or HCT. A 2022 Nature article (Nature 2022, vol 610, 783: https://www.nature.com/articles/s41586-022-05312-w) had suggested that Roxadustat could induce fetal hemoglobin at the cellular level, indicating its potential use for SCD. However, this experiment shows that the performance of Roxadustat in the SCD mouse model is completely unexpected, as Roxadustat had no therapeutic effect in SCD mice whatsoever.

Daprodustat mildly improved RBC, Hb, and HCT. At the end of the four-week experiment, RBC increased from 6.14 M/µL to 7.54 M/µL, Hb increased from 9.1 g/dL to 10.9 g/dL, and HCT increased from 31.8% to 35.9%. One mouse in the Daprodustat group including six mice in total died after two weeks of dosing, so the baseline data was the average of six mice, while the four-week endpoint data was the average of five mice. This result indicates that Daprodustat had a mild effect in the SCD mouse model, but it was far inferior to Comp-1.

From the above results, it can be seen that although EPO is known to be usable for anemia such as CKD anemia, it has no therapeutic effect in the SCD mouse model. Roxadustat can induce fetal hemoglobin at the cellular level but is completely ineffective in the SCD mouse model. Daprodustat can mildly improve RBC, Hb, and HCT. However, Comp-1 demonstrated surprising efficacy.

Although embodiments of the present application have been illustrated and described, it is not intended that these examples illustrate and describe all possible forms of the present application. Rather, the words used in the specification are words of description rather than limitation, and it is understood that various changes may be made without departing from the spirit and scope of the present application.

## Claims

1. Use of a hypoxia-inducible factor-prolyl hydroxylase inhibitor (HIF-PHI) in the manufacture of a medicament for treating a rare anemia.

2. Use of a hypoxia-inducible factor-prolyl hydroxylase inhibitor (HIF-PHI) in the manufacture of a medicament for increasing red blood cell count, hemoglobin levels, and/or hematocrit in a rare anemia.

3. A method for treating a rare anemia, comprising administering to a patient in need thereof a therapeutically effective amount of a hypoxia-inducible factor-prolyl hydroxylase inhibitor (HIF-PHI).

4. A method for increasing red blood cell count, hemoglobin levels, and/or hematocrit in a rare anemia, comprising administering to a patient in need thereof a therapeutically effective amount of a hypoxia-inducible factor-prolyl hydroxylase inhibitor (HIF-PHI).

5. The use or method according to any one of claims 1 to 4, wherein the rare anemia is selected from myelodysplastic syndrome-associated anemia (MDS anemia), β-thalassemia, or sickle cell disease (SCD anemia).

6. The use or method according to any one of claims 1 to 4, wherein the hypoxia-inducible factor-prolyl hydroxylase inhibitor (HIF-PHI) is Comp-1.

7. The use or method according to any one of claims 1 to 4, wherein the HIF-PHI is a compound of Formula I, or a pharmaceutically acceptable salt or tautomer thereof: 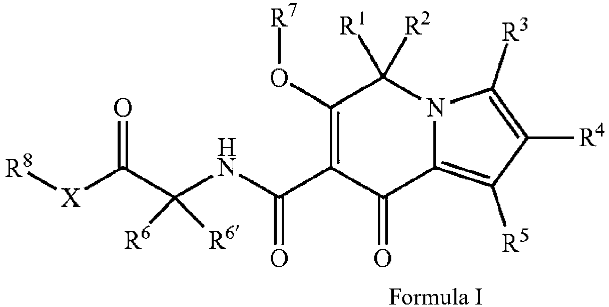 wherein:
R¹ and R² are independently selected from the group consisting of cyano, alkyl, heterocyclyl, alkenyl, alkynyl, aryl, heteroaryl, and acyl; wherein the aforementioned alkyl, heterocyclyl, alkenyl, alkynyl, aryl, heteroaryl, and acyl are optionally substituted by one or more substituents independently selected from the group consisting of halogen, cyano, hydroxyl, amino, carboxyl, acyl, alkyl, heterocyclyl, alkenyl, alkynyl, aryl, heteroaryl, =O, =S, SH, R¹⁰O-, R¹⁰S-, R¹⁰ (O=)S-, and R¹⁰ (O=)₂S-, wherein R¹⁰ is alkyl, heterocyclyl, alkenyl, alkynyl, aryl, or heteroaryl; or R₁ and R₂ are taken together to form a ring;
R³ is selected from the group consisting of alkyl, heterocyclyl, alkenyl, alkynyl, aryl, and heteroaryl;
R⁴ and R⁵ are hydrogen;
R⁶ and R⁶' are hydrogen;
R⁷ is hydrogen;
R⁸ is selected from the group consisting of hydrogen and alkyl; and
X is an oxygen atom.

8. The use or method according to claim 7, wherein in the compound of Formula I, R¹ and R² together with a carbon atom to which they are attached form a cyclopentane ring (i.e., R¹ and R² are -CH₂CH₂CH₂CH₂-), R³ is phenyl, R⁴ and R⁵ are hydrogen, R⁶ and R⁶' are hydrogen, R⁷ is hydrogen, R⁸ is hydrogen, and X is an oxygen atom, that is, the compound of Formula I is (6'-hydroxy-8'-oxo-3'-phenyl-8'H-spiro[cyclopentane-1,5'-indolizine]-7'-carbonyl)glycine.

9. The use or method according to any one of claims 1 to 4, wherein when the rare anemia is myelodysplastic syndrome-associated anemia (MDS anemia), the HIF-PHI is selected from the group consisting of Daprodustat, Vadadustat, Molidustat, Enarodustat, Desidustat, HIF117 (SSS17), HEC53856, DDO-3055, and FG-2216.

10. The use or method according to any one of claims 1 to 4, wherein when the rare anemia is β-thalassemia, the HIF-PHI is selected from the group consisting of Roxadustat, Daprodustat, Vadadustat, Molidustat, Enarodustat, Desidustat, HIF117 (SSS17), HEC53856, DDO-3055, and FG-2216.

11. The use or method according to any one of claims 1 to 4, wherein when the rare anemia is sickle cell disease (SCD anemia), the HIF-PHI is selected from the group consisting of Daprodustat, Vadadustat, Molidustat, Enarodustat, Desidustat, HIF117 (SSS17), HEC53856, and DDO-3055.
